Europäisches Patentamt

European Patent Office

Office européen des brevets

(11) Publication number : **0 616 842 A1**

# (12) EUROPEAN PATENT APPLICATION

(21) Application number : **94301778.0**

(22) Date of filing : **11.03.94**

(51) Int. Cl.$^5$ : **B01J 13/00, C08K 5/09**

(30) Priority : **17.03.93 US 32080**

(43) Date of publication of application :
**28.09.94 Bulletin 94/39**

(84) Designated Contracting States :
**DE FR GB**

(71) Applicant : **DOW CORNING CORPORATION**
**3901 S. Saginaw Road**
**Midland Michigan 48686-0994 (US)**

(72) Inventor : **Katsoulis, Dimitris Elias**
**2202 Wilmington Drive**
**Midland, Michigan (US)**
Inventor : **Bujanowski, Valerie Joy**
**318 Norfolk Drive**
**Midland, Michigan (US)**
Inventor : **Ziemelis, Maris Jazeps**
**2015 W. Nelson**
**Midland, Michigan (US)**
Inventor : **Weiss, Richard Gerald**
**4604 West Virginia Avenue**
**Bethseda, Maryland (US)**

(74) Representative : **Laredo, Jack Joseph et al**
**Elkington and Fife**
**Prospect House**
**8 Pembroke Road**
**Sevenoaks, Kent TN13 1XR (GB)**

(54) **Silicone gels and methods of preparing the same.**

(57) Gels, stable at a predetermined service temperature and formed from silicone solvent fluids and a solute gelator, are disclosed. The solute gelator is selected from stearic acid ; 12-hydroxystearic acid ; metal salts of 12-hydroxy- stearic acid ; metal salts of stearic acid and admixtures thereof. The solute gelator is dissolved in the silicone solvent and forms a solution therewith that is supersaturated at the predetermined service temperature. The gelator is then precipitated from the solution, forming a three-dimensional fibrous network that imparts sufficient mechanicl properties to the silicone solvent to form a gel.

EP 0 616 842 A1

The present invention relates to gelling silicone fluids and more specifically to the formation of such gels wherein stearic acid, 12-hydroxystearic acid or a metal salt of either compound, is used as a gelator.

The formation of gels from fluids such as organic solvents is known in the art; see, for example, U.S. Patent No. 4,790,961 which discloses the formation of thermally reversible gels from organic solvents and a gelator of the general formula $R_1$-$(CH_2)_n$-$CO_2$-$R_2$ wherein $R_1$, $\underline{n}$ and $R_2$ are defined therein. While this patent indicates that silicone oil is included in the general class of organic solvents, no specific examples of silicone gelation are disclosed.

Other materials which form gels when combined with organic solvents have also been taught. Tachibana et al. (Vol. 42, No. 12, Bulletin Of The Chemical Society of Japan, pp. 3422 - 3424 [1969]) report the formation of gels from various organic solvents (including mineral oil and ethanol) by utilizing 12-hydroxystearic acid and its lithium salt as gelling agents.

While this prior art discloses gelling organic solvents, there has long existed a need for a simple means for gelling silicones. Gelled silicones are very desirable for use in the manufacture of lubricants and cosmetic vehicles.

An ideal gelator should work in low concentration so as not to interfere with the desirable physical and chemical properties of the silicone. Furthermore, the gelator should not interfere with the intended use of the gelled silicone. For example, a gelator that is toxic would have little or no value for cosmetic formulations and vehicles.

Finally, the silicone gel formed in conjunction with the gelator should be stable. As used herein, the term "stable" means that, at a predetermined service temperature, the silicone fluid will not leach or separate from the gel under the influence of gravity. Accordingly, a stable silicone gel will remain in gel form indefinitely if protected from chemical contamination, mechanical shock and evaporation loss.

The present invention provides a gelled silicone solvent comprising a silicone solvent in combination with a gel-forming amount of a solute gelator. The gelled silicone solvent is stable at a predetermined service temperature and the solute gelator has limited solubility in the silicone solvent at the predetermined service temperature. In its untreated state, the silicone solvent is normally fluid at the predetermined service temperature. The solute gelator is selected from stearic acid; 12-hydroxystearic acid; metal salts of stearic acid; metal salts of 12-hydroxystearic acid; and admixtures thereof.

In the method of the present invention, the silicone solvent is gelled by steps comprising:

(a) forming a solution of the solute gelator in the silicone solvent, the solution being supersaturated with the gelator at the predetermined service temperature and the gelator being selected from stearic acid 12-hydroxystearic acid, metal salts of stearic acid; metal salts of 12-hydroxystearic acid, and admixtures thereof; and

(b) precipitating the gelator from the solution.

Upon such precipitation, the gelator forms a three-dimensional fibrous network in the silicone solvent. This fibrous network is stable at the predetermined service temperature and imparts sufficient mechanical strength to the silicone solvent so as to form a gel therefrom.

The solution-forming step may be carried out by heating a mixture of the silicone solvent and solute gelator to a temperature in excess of the predetermined service temperature. The precipitation step is then carried out by cooling the solution back to the service temperature, whereupon the gel forms.

Alternatively, the solution-forming step may be carried out at or near the predetermined service temperature by first dissolving the solute gelator in a co-solvent, which is then introduced into the silicone solvent. Because the silicone solvent solution is then supersaturated with solute gelator, the gelator precipitates, forming the gel of the invention.

Those skilled in the art will recognize that the service temperature for the gelled silicone can vary, depending upon its application. For instance, if the gelled silicone solvent is to be used as a vehicle for antiperspirant salts or cosmetics, the predetermined service temperature is generally considered to be room temperature. On the other hand, if the gelled silicone solvent is to be used as a moisture barrier in underground cable applications, the predetermined service temperature is substantially lower. Finally, it will be further recognized that the term "predetermined service temperature" can encompass a fairly broad temperature range, within which the gelled silicone solvent may be used.

As used herein the term "silicone solvent" means any of a large group of siloxane polymers based upon a structure consisting of alternating silicon and oxygen atoms with various organic radicals attached to the silicon atoms. The polymers may be linear, branched or cyclic. Typical organic radicals include hydrogen and alkyl, aryl and alkenyl groups. The siloxane polymers may also include reactive organofunctional groups such as carbinol, amine, alkoxy and epoxy groups, so long as the reactive organofunctional radicals do not prevent the formation of a stable gel in accordance with the invention. Such siloxane polymers are well-known in the art.

In order to practise the present invention however, two additional criteria with respect to the silicone solvent must be met: (1) in its untreated state the silicone solvent must be fluid at the predetermined service temperature; and (2) the solute gelator of the invention must have limited solubility in the silicone solvent at the predetermined service temperature. For predetermined service temperatures in the range of room temperature, silicone solvents having a number average molecular weight between about 164 and about 150,000 are generally considered fluid.

We have experimented with, and developed, several silicone solvents and various mixtures as solute gelators. Among them are stearic acid; 12-hydroxystearic acid; metal salts of stearic acid; metal salts of 12-hydroxystearic acid; and admixtures thereof. In addition, various analogues of 12-hydroxy stearic acid were tested for their efficacy as solute gelators.

Two basic types of silicone solvents were used: trimethylsilyl end-blocked polydimethylsiloxane fluids of various viscosities (hereinafter referred to as "PDMS"); and a mixture of cyclic siloxanes which comprised: 24 weight % dodecamethylcyclohexasiloxane; 68 weight % decamethylcyclopentasiloxane; 6 weight % octamethylcyclotetrasiloxane; and the balance impurities (hereinafter referred to simply as "cyclics").

Reported below in Table 1 is a list of silicone fluids and mixtures that were successfully gelled with a 1 weight % addition of 12-hydroxystearic acid as the solute gelator. In each instance, the 12-hydroxystearic acid was dissolved in an ethanol co-solvent, the use of which is explained in detail below. The gelator/ethanol solution was added directly to a small quantity (less than 2 g) of the silicone fluid. The mixture was then heated to approximately 80°C in an oil bath, whereupon substantially all the ethanol was volatilized and at least a portion of the 12-hydroxystearic acid solute dissolved in the silicone solvent fluid. The solutions so-formed were then pipetted into small glass vials and allowed to cool. Upon cooling, the gelator precipitated from the silicone solvent and formed a fibrous network therein. It is believed that the fibrous network traps the silicone fluid, which results in the formation of the silicone solvent gel. The appearance of each gel was noted, as reported below.

## TABLE 1

### SILICONE SOLVENT FLUIDS GELLED WITH 1 WEIGHT % 12-HYDROXYSTEARIC ACID

| EX. | FLUID/FLUID MIXTURE | OBSERVATIONS |
|---|---|---|
| 1 | cyclics | opaque, stable |
| 2 | PDMS (0.65) | opaque, stable |
| 3 | PDMS (1.0) | opaque, stable |
| 4 | PDMS (10) | opaque, stable |
| 5 | PDMS (100) | clear, stable |
| 6 | PDMS (350) | clear with areas of large non-fibrous crystals, stable |
| 7 | PDMS (350, 0.45 wt% 12-OH stearic acid) | clear with areas of small non-fibrous crystals, stable |
| 8 | cyclics/PDMS (100) (66/44**) | translucent, stable |
| 9 | cyclics/PDMS (5,000) (85/15) | translucent, stable |
| 10 | cyclics/PDMS (350) (50/50) | slightly hazy, very small non-fibrous crystals, stable |

* viscosity in $mm^2$/s (cs) at 25°C.

** parts, by weight, respectively

From Table 1 it can be seen that by blending different silicone fluids, the physical properties of the gel which is ultimately formed therefrom can be varied.

Furthermore, as the solution formed from the silicone solvent and the solute gelator cools to room temperature, the solubility of the gelator in the solvent decreases and the gelator precipitates out, forming the fibrous network. In the examples set forth in Table 1, the predetermined service temperature was room temperature. Thus, the solution formed between the silicone solvent and the solute gelator would have been supersaturated at the predetermined service temperature. In each instance, the fibrous network imparted sufficient mechanical properties to the silicone solvent to form a stable gel therefrom.

In general, the data in Table 1 show that, as the viscosity of the silicone solvent increases, the clarity of the gel formed therefrom also increases. Because the viscosity of a silicone fluid or admixtures thereof is directly related to mean molecular weight, gel clarity is also related thereto.

The present inventors have studied the morphology of the fibrous precipitate formed in the silicone fluid and have found that it is also related to the viscosity and hence the clarity of the gel. In general, as the mean molecular weight of a silicone fluid increases, the diameters of the precipitate fibrils formed from the solute gelator decrease. At the same time, the fibril population density increases. In relatively low molecular weight silicone solvents (number average molecular weights of 1050 and less, which in PDMS corresponds to a viscosity of 10 mm$^2$/s (centistokes) or less at 25°C.), fibril diameters are typically 500 nanometers. In relatively high molecular weight silicone solvents (number average molecular weights of 4000 and above, which in PDMS corresponds to a viscosity of 100 mm$^2$/s (centistokes) and above), fibril diameters are typically 50 nanometers or less. It is believed that the finer fibril dimensions and the denser fibril population are responsible for imparting more clarity to the gelled silicone solvent.

Referring now to Table 1 and specifically to Examples 6 and 7 and the observations associated therewith, it can be seen that the solute gelator precipitated from the silicone solvent in two different forms: the fibrous network that formed the stable gel; and in the form of non-fibrous crystals. It can also be seen that,when the concentration of the solute gelator is reduced, the size of the non-fibrous crystals is reduced.

The present inventors have discovered that a co-solvent can be useful for dissolving the solute gelator in the silicone solvent. As used herein, the term "co-solvent" means: a solvent in which the solute gelator has a greater solubility than in the silicone solvent; and a solvent that is itself soluble in the silicone solvent.

Accordingly, the co-solvent can be used to deliver the solute gelator into the silicone solvent at the predetermined service temperature. In such a delivery system, the solute gelator may be introduced in such a concentration that the silicone solvent is supersaturated upon mixing. The gelator then precipitates from the silicone solvent forming the previously described fibrous network, thus gelling the silicone solvent at the predetermined service temperature.

It is preferable to use a co-solvent that is miscible with the silicone solvent and in which the gelator has a high degree of solubility. In such a system, the amount of gelator that is introduced into the silicone solvent can be readily controlled.

The use of a co-solvent may also be combined with a heating step to form the silicone solvent gels of the invention. An ideal co-solvent for such an application is ethanol. Ethanol readily dissolves the solute gelators of the invention. When the use of an ethanol co-solvent is combined with a heating step, the ethanol is readily driven off, leaving substantially pure silicone solvent and solute gelator in the system.

It was speculated that the gelled silicones of the invention would have utility in the formation of antiperspirant sticks. In order to determine the feasibility of such use, the aforementioned cyclics were combined with 20 weight % granulated aluminum chlorohydrate (impalpable grade, 100% -37 micrometer [-400 Tyler mesh]). This composition was gelled using 1 weight % 12-hydroxystearic acid. A stable gel was formed with no deleterious effects on the antiperspirant powder being observed.

The terms used to describe the relative clarity or opacity of the various gels of the invention are somewhat subjective in nature. Clarity may, however, be measured by determining the amount of visible light absorbed by a given thickness of a gel sample when an incident beam of known wavelength and intensity is passed therethrough.

Several samples of silicone solvent gels were prepared by heating a mixture of one gram of silicone solvent having 1 weight % of 12-hydroxystearic acid added thereto. The mixtures were heated in a glass vial immersed in an oil bath that was maintained between 84°C. and 90°C. The heated mixtures were then agitated to ensure good mixing between the solute gelator and the silicone fluid solvent and the solution was pipetted into a reservoir formed between a pair of spaced-apart, parallel glass plates. The solution in the reservoir was permitted to cool to room temperature on the laboratory bench, which resulted in the formation of a silicone gel.

In this manner, several gel specimens, one mm thick, were formed in the reservoir between the spaced-apart glass plates. The relative clarity of the gels was observed and the plates placed in a specimen holder. A spectro-photometer was then used to measure the amount of light absorbed by the specimen when incident beams of light (600 and 450 nm wave-lenths) were passed through the 1 mm thick gel sections. The absorbed light was reported as a fraction of the intensity of the incident beam. The light absorption results for the various silicone solvent gels (and a 1 weight % solution of 12-hydroxystearic acid in 200 proof ethanol, as a control) are reported below in Table 2.

## TABLE 2

### SILICONE SOLVENT GEL LIGHT ABSORPTION

| EX. | SILICONE SOLVENT | ABSORPTION | | OBSERVATION |
|---|---|---|---|---|
| | | 600 nm | 450 nm | |
| 1 | ethanol* | 0.0 | 0.0 | clear solution |
| 2 | cyclics | 0.080 | 0.103 | translucent gel |
| 3 | PDMS (0.65**) | 0.154 | 0.247 | translucent to slightly opaque gel |
| 4 | PDMS (100) | 0.130 | 0.130 | translucent gel |
| 5 | PDMS (10) | 0.109 | 0.102 | translucent gel |
| 6 | Ex. 5 w/3 drops ethanol | 0.766 | 0.827 | opaque gel |

*control
**viscosity in $mm^2/s$ (cs) at 25°C.

The data presented in Table 2 show that the term "clear," as applied to gels and the like, can be represented by an absorption of 0.0, i.e., no absorption of the incident beam. Any value in excess of 0.0 is then not entirely "clear." On the other hand, gels having absorption values of 0.05 or less are generally perceived as "clear." Likewise, gels having absorption values in excess of 0.05 are generally perceived as translucent and those having absorption values in excess of 0.3 are generally perceived as opaque. Thus, as used herein to describe silicone solvent gels, the terms "clear," "translucent" and "opaque" mean perceptibly clear, translucent and opaque.

A comparison of Examples 5 and 6 in Table 2 reveals that the use of ethanol as a co-solvent in the present invention can strongly influence the opacity of the gel formed from the silicone solvent. Thus, if clarity is desired the use of an ethanol co-solvent should be avoided or a heating step sufficient to volatilize the ethanol should be combined therewith. However, volatilizing a sufficient quantity of the ethanol to obtain a clear gel can prove difficult.

It has been found that the silicone solvent gels of the invention are thermally reversible. That is to say, when a gel of the invention is heated to a temperature at which the solubility of the solute gelator in the silicone solvent causes redissolution of the gelator, the fluid consistency of the silicone solvent returns. Upon cooling, however, the gel reforms. This property is important to note as the manufacture of antiperspirant sticks usually involves casting a molten composition in molds.

The melting point of the solute gelators of the present invention can vary. Relatively pure 12-hydroxystearic acid has a melting point of 79.6°C. Its sodium salt, prepared by neutralization with NaOH, has a melting point of 150.7°C. Regardless of the solubility of the gelator in the silicone solvent, at temperatures at and above the melting point of the gelators, the fluid characteristics of the ungelled silicone solvent return.

Several attempts were made to determine whether any chemical analogues of 12-hydroxystearic acid would form stable gels with silicone solvents and with silicone solvents admixed with various organic fluids. Table 3, below, shows a number of carboxylic acids which were added to the silicone fluids and admixtures.

TABLE 3

GELLING EFFICACY OF 2 WEIGHT % OF VARIOUS
CARBOXYLIC ACIDS IN SILICONE
FLUIDS AND SILICONE FLUID ADMIXTURES

| EX. | CARBOXYLIC ACID | SILICONE SOLVENT/ ADMIXTURE | OBSERVATIONS |
|---|---|---|---|
| 1 | DL-2-hydroxy-3-butyric acid | cyclic | no gel, white precipitate |
| 2 | 2-hydroxyoctanoic acid | cyclic | unstable[a] gel |
| 3 | 2-hydroxyoctanoic acid | PDMS (350*) | no gel |
| 4 | 2-hydroxyoctanoic acid | PDMS (350) /cyclic | unstable[a] opaque gel |
| 5 | 2-hydroxyoctanoic acid | PDMS (100) | thickened fluid no gel |
| 6 | 11-hydroxyundecanoic acid | cyclic | no gel, white precipitate |
| 7 | 11-hydroxyundecanoic acid | cyclic /ethanol | partial[b] gel |
| 8 | 2-hyroxytetradecanoic acid | cyclic | no gel, white precipitate |
| 9 | 2-hyroxypentadecanoic acid | cyclic | no gel, white precipitate |
| 10 | 2-hyroxyhexadecanoic acid | cyclic | no gel, white precipitate |
| 11 | stearic acid[c] | cyclic | unstable[a] gel |
| 12 | stearic acid | PDMS (350) /mineral oil | stable opaque gel |

[a] the gel was disrupted after 24 hours

[b] partial gelation occurred on surface, after gelation

[c] acid concentration = 4 %

*viscosity in $mm^2/s$ (cs) at 25°C.

Table 3 shows, surprisingly, that, of the chemical analogues of 12-hydroxystearic acid that were tested, only stearic acid produced a stable gel. Without limiting the present invention, it is theorized that the non-gel-forming analogues failed to precipitate in the form of a fibrous network capable of imparting sufficient mechanical properties to result in a stable gel.

Finally, it should be noted from Table 2, example 6 and Table 3, example 12, that,even if organic solvents are mixed with silicone solvents, the silicone solvent gels of the invention may still be formed. The term "organic solvent" as used herein has the same meaning as set forth in U.S. Patent No. 4,790,961.

EP 0 616 842 A1

**Claims**

1. A method of forming a gel from a silicone solvent, which method comprises the steps of:
   (a) forming a solution of a solute gelator in a silicone solvent,
      said solution being supersaturated with said gelator at a predetermined service temperature, and
      said gelator being selected from stearic acid; 12-hydroxystearic acid; metal salts of stearic acid; metal salts of 12-hydroxystearic acid; and admixtures thereof; and
   (b) precipitating said gelator from said supersaturated silicone solvent, said gelator thereby forming a three-dimensional fibrous network in said silicone solvent,
      said fibrous network being stable at said predetermined service temperature; and
      said fibrous network imparting sufficient mechanical strength to said silicone solvent so as to form a gel therefrom, whereby a gel is formed which is stable at a predetermined service temperature, said silicone solvent being fluid at said predetermined service temperature in its untreated state.

2. A method in accordance with claim 1, wherein said solution-forming step (a) is carried out by heating said silicone solvent to a temperature in excess of said predetermined service temperature to dissolve said gelator therein; and
      said precipitating step (b) is carried out by cooling said supersaturated solution to cause precipitation of said gelator therefrom.

3. A method in accordance with claim 1 wherein said solution-forming step is carried out by first dissolving said gelator in a co-solvent; and then mixing said co-solvent having said gelator dissolved therein with said silicone solvent.

4. A method in accordance with claim 3 wherein said mixing step is carried out at the predetermined service temperature.

5. A method in accordance with claim 2 wherein said solution-forming step comprises:
      mixing a co-solvent with said silicone solvent and said gelator; and
      heating the mixture so-formed to a temperature above the melting point of said gelator.

6. A method in accordance with claim 5 wherein said heating step causes said co-solvent to volatilize.

7. A method in accordance with claim 1 wherein said silicone solvent is selected from silicone cyclics, polydimethylsiloxane and admixtures thereof.

8. A method in accordance with claim 1 wherein said silicone solvent has a mean molecular weight in the range of from 164 to 150,000, inclusive.

9. A method in accordance with claim 1, wherein the silicone gel that is formed is clear.

10. A method in accordance with claim 1, further comprising the step of adding a granular antiperspirant to said silicone solvent, prior to carrying out said precipitating step.

11. A method in accordance with claim 1, wherein said predetermined service temperature is room temperature.

12. A silicone solvent gel comprising:
      a silicone solvent which, in its untreated state, is fluid at a predetermined service temperature;
      a gel-forming quantity of a solute gelator,
      said solute gelator being selected from stearic acid; 12-hydroxy stearic acid; metal salts of stearic acid; metal salts of 12-hydroxystearic acid; and admixtures thereof, and
      said silicone solvent gel being stable at said predetermined service temperature.

13. A silicone solvent gel in accordance with claim 12, wherein said gel is perceptibly clear.

14. A silicone solvent gel in accordance with claim 12, wherein said gel is perceptibly translucent.

15. A silicone solvent gel in accordance with claim 12, wherein said gel is perceptibly opaque.

7

16. A silicone solvent gel in accordance with claim 12, wherein said gel-forming quantity of said solute gelator is less than 4 weight percent of said gel.

17. A silicone solvent gel in accordance with claim 12, further comprising at least one organic solvent.

18. A silicone solvent gel in accordance with claim 12, further comprising a granular antiperspirant salt dispersed therein.

**European Patent Office**

## EUROPEAN SEARCH REPORT

Application Number

EP 94 30 1778

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X,P | PATENT ABSTRACTS OF JAPAN vol. 17, no. 462 (C-1101) 24 August 1993 & JP-A-05 112 424 (KAO CORP) 7 May 1993 * abstract * | 1,12 | B01J13/00 C08K5/09 |
| A | GB-A-2 253 347 (DOW CORNING) | | |
| A | FR-A-1 261 155 (FARBENFABRIKEN BAYER) | | |
| A,D | WO-A-88 00938 (GEORGETOWN UNIVERSITY) | | |
| A,D | BULLETIN OF THE CHEMICAL SOCIETY OF JAPAN, vol.42, no.12, 1969 pages 3422 - 3424 TARO TACHIBANA ET AL. 'STUDIES OF HELICAL AGGREGATES OF MOLECULES' | | |

**TECHNICAL FIELDS SEARCHED (Int.Cl.5)**

B01J
C08K

The present search report has been drawn up for all claims

| Place of search | Date of completion of the search | Examiner |
|---|---|---|
| THE HAGUE | 30 June 1994 | Meertens, J |

EPO FORM 1503 03.82 (P04C01)